# EUROPEAN PATENT APPLICATION

(11) **EP 2 204 644 A1**
(43) Date of publication of application: **07.07.2010**
(21) Application number: 08834400.7
(22) Date of filing: 17.09.2008
(51) Int. Cl.: G01N 19/04

(54) **CROSSCUT TESTING METHOD, AND CROSS-CUT TESTING DEVICE**

(30) Priority: 27.09.2007 JP 2007251588; 28.02.2008 JP 2008047589
(71) Applicant: Daikin Industries, Ltd., Osaka-shi, Osaka 530-8323 (JP)
(72) Inventor: INOMOTO, Hisashi, Sakai-shi Osaka 591-8511 (JP); OHKAWA, Takeyoshi, Sakai-shi Osaka 591-8511 (JP)
(74) Representative: HOFFMANN EITLE
(86) International application number: PCT/JP2008/002555
(87) International publication number: WO 2009/040999

(57) **Abstract**

In a cross-cut test, after cuts (12) are vertically and horizontally formed in a coating film (11) applied to a base material (10) to make a peeling force act on the coating film (11), adhesion of the coating film (11) is evaluated based on a peeling state of the coating film (11). In the cross-cut test, first vertical cuts (12a) are formed at regular or irregular intervals, and intervals between second horizontal cuts (12b) are gradually narrowed, in order to quantitatively measure the adhesion of the coating film (11) to the base material (10), and to reduce complicated working processes.

## Description

### TECHNICAL FIELD

The present invention relates to device and method for obtaining quantitative measurement results in a cross-cut test for measuring coating adhesion to a base material.

### BACKGROUND ART

A cross-cut test (cross-cut technique) according to Japanese Industrial Standards (JIS) K 5600-5-6 is a test where, after horizontal and vertical cuts are formed at regular intervals in a coating film applied on a base material, an adhesive tape is adhered on and removed from the surface, thereby determining coating adhesion depending on the number of peeled cells.

For example, Patent Document 1 discloses that a coating on a base material is cut in a grid pattern, and a jig is bonded to each of the formed cells to apply tensile stress, thereby evaluating adhesion strength of the coating based on its peeling load.

### CITATION LIST

### PATENT DOCUMENT

PATENT DOCUMENT 1: Japanese Patent Publication No. 06-16744

### SUMMARY OF THE INVENTION

### TECHNICAL PROBLEM

The cross-cut test generally determines the coating adhesion to the base material based on whether or not the coating film is peeled off, and therefore it is difficult to quantitatively measure the coating adhesion. In addition, as for material such as fluororesin to which an adhesive tape is hardly adhered, and non-flat material, it is difficult to remove the tape with good reproducibility.

In Patent Document 1, it is necessary to bond the jig to the base material, resulting in the increased number of working processes. Consequently, the measurement cannot be easily performed.

The present invention has been made in view of the foregoing, and it is an object of the present invention to quantitatively measure the coating adhesion to the base material in the cross-cut test, and to reduce the complicated working processes.

### SOLUTION TO THE PROBLEM

A first aspect of the invention is intended for a cross-cut testing method sequentially performing a cut forming process for vertically and horizontally forming cuts (12) in a coating film (11) applied to a base material (10); a peeling process for making a peeling force act on the coating film (11) after the formation of the cuts (12); and an evaluating process for evaluating adhesion of the coating film (11) based on a peeling state of the coating film (11) after the peeling process.

The cut forming process of the cross-cut testing method is characterized by forming first cuts (12a) in one of the groups of vertical and horizontal cuts at regular or irregular intervals; and forming second cuts (12b, 12c) in the other group of vertical and horizontal cuts at irregular intervals. The peeling process may be a process performed by using adhesive tapes, etc., or may be a process in which the intervals between the cuts (12) are narrowed, resulting in the coating film (11) being naturally peeled off by the peeling force acting between such cuts (12). That is, the peeling process may be performed at approximately the same time as the cut forming process. If the thickness of the coating film (11) is nonuniform at each evaluation, the adhesion is changed. Thus, it is preferred that the thickness of the coating film (11) is uniform.

In the first aspect of the invention, a narrower interval between the cuts (12) generates weaker adhesion of the coating film (11) to the base material (10). The coating film (11) tends to be peeled off in a portion having a narrower interval between the cuts (12) as compared to a portion having a wider interval between the cuts (12). Thus, the intervals between the cuts (12), at which cells of the coating film (11) are peeled off, are evaluated, thereby determining the adhesion of the coating film (11).

A second aspect of the invention is intended for the cross-cut testing method of the first aspect of the invention, in which the cut forming process includes forming the first cuts (12a) at regular intervals, and forming the second cuts (12b, 12c) at irregular intervals.

In the second aspect of the invention, a narrower interval between the second cuts (12b, 12c) generates weaker adhesion of the coating film (11) to the base material (10). The coating film (11) tends to be peeled off in a portion having a narrower interval between the second cuts (12b, 12c) as compared to a portion having a wider interval between the second cuts (12b, 12c). Thus, the intervals between the second cuts (12b, 12c), at which cells of the coating film (11) are peeled off, are evaluated, thereby determining the adhesion of the coating film (11).

A third aspect of the invention is intended for the cross-cut testing method of the first or second aspect of the invention, in which the cut forming process includes forming the second cuts (12b, 12c) so that the intervals are gradually increased or decreased from one end to the other end.

In the third aspect of the invention, the intervals between the second cuts (12b, 12c) are gradually changed from one end to the other end. If narrow and wide intervals between the second cuts (12b, 12c) are mixed, peeled portions may be also scattered. However, in the present invention, the peeling is concentrated in portions having smaller intervals between the second cuts (12b, 12c).

A fourth aspect of the invention is intended for a cross-cut testing device for performing a cross-cut test for a coating film (11), which includes a cut forming mechanism (20) for vertically and horizontally forming cuts (12) in the coating film (11) applied to a base material (10); and a peeling mechanism (20) for making a peeling force act on the coating film (11) in which the cuts (12) are formed.

In addition, the cut forming mechanism (20) of the cross-cut testing device is characterized by including first cutting means (20a) for forming first cuts (12a) in one of the groups of vertical and horizontal cuts at regular or irregular intervals; and second cutting means (20b) for forming second cuts (12b, 12c) in the other group of vertical and horizontal cuts at irregular intervals. In such a structure, the peeling mechanism (20) may be a mechanism for purposely peeling the coating film (11) by using adhesive tapes, etc., or may be a mechanism using a peeling force which naturally acts on the coating film (11) between the cuts (12) by narrowing the intervals by the cut forming mechanism (20) (i.e., a structure in which the cut forming mechanism (20) also serves as the peeling mechanism (20)).

In the fourth aspect of the invention, a narrower interval between the cuts (12) formed by the cut forming mechanism (20) generates weaker adhesion of the coating film (11) to the base material (10). The coating film (11) tends to be peeled off in a portion having a narrower interval between the cuts (12) as compared to a portion having a wider interval between the cuts (12). Thus, the intervals between the cuts (12), at which cells of the coating film (11) are peeled off, are evaluated, thereby determining the adhesion of the coating film (11).

A fifth aspect of the invention is intended for the cross-cut testing device of the fourth aspect of the invention, in which the first cutting means (20a) forms the first cuts (12a) at regular intervals.

In the fifth aspect of the invention, a narrower interval between the second cuts (12b, 12c) formed by the cut forming mechanism (20) generates weaker adhesion of the coating film (11) to the base material (10). The coating film (11) tends to be peeled off in a portion having a narrower interval between the second cuts (12b, 12c) as compared to a portion having a wider interval between the second cuts (12b, 12c). Thus, the intervals between the second cuts (12b, 12c), at which cells of the coating film (11) are peeled off, are evaluated, thereby determining the adhesion of the coating film (11).

A sixth aspect of the invention is intended for the cross-cut testing device of the fourth or fifth aspect of the invention, in which the second cutting means (20b) forms the second cuts (12b, 12c) in the coating film (11) so that the intervals are gradually increased or decreased from one end to the other end.

In the sixth aspect of the invention, the intervals between the second cuts (12b, 12c) are gradually changed from one end to the other end. If narrow and wide intervals between the second cuts (12b, 12c) are mixed, peeled portions may be also scattered. However, in the present invention, the peeling is concentrated in portions having smaller intervals between the second cuts (12b, 12c).

A seventh aspect of the invention is intended for the cross-cut testing device of the fifth or sixth aspect of the invention, in which the cut forming mechanism (20) is intended to form the cuts in the coating film (11) formed on the cylindrical base material (10); the first cutting means (20a) forms the first cuts (12a) in an axial direction at regular intervals; and the second cutting means (20b) forms the second cuts (12b, 12c) in a circumferential direction at irregular intervals.

A eighth aspect of the invention is intended for the cross-cut testing device of the seventh aspect of the invention, in which the second cutting means (20b) forms a plurality of second cuts (12b) which are continuous in the circumferential direction, and which are discontinuous in the axial direction.

A ninth aspect of the invention is intended for the cross-cut testing device of the seventh aspect of the invention, in which the second cutting means (20b) forms a second cut (12c) which is a single spiral continuous in the circumferential and axial directions, and a torsion angle of which is gradually changed.

In the seventh to ninth aspects of the invention, e.g., if a coating having smaller friction resistance is formed on a sliding bearing or a bush as machine elements, adhesion of such a coating can be determined based on the first cuts (12a) formed at regular intervals in the axial direction, and the second cuts (12b, 12c) formed at irregular intervals in the circumferential direction. If the cross-cut test of the present invention is performed on the cylindrical base materials (10) as described above for relative comparison, it is preferred that shapes (inner diameters) of the cylinders are identical.

### ADVANTAGES OF THE INVENTION

According to the present invention, the intervals between the cuts (12) formed in the coating film (11) applied to the base material (10) are differentiated, thereby changing the tendency of the coating film (11) to be peeled off (adhesion). Consequently, the coating film (11) tends to be peeled off in the portion having the narrower interval between the cuts (12) as compared to the portion having the wider interval between the cuts (12). Thus, the intervals between the cuts (12), at which the cells of the coating film (11) are peeled off, are evaluated, thereby quantitatively determining the adhesion of the coating film (11). Consequently, the adhesion of the coating film (11) can be more accurately evaluated as compared to a conventional way. In addition, in the present embodiment, it is not necessary to adhere jigs on the base material (10), resulting in a simple measurement. Further, in the present invention, the adhesion of the coating film (11) can be determined without using adhesive tapes. Thus, the present invention is suitable for using material with poor adhesion, e.g., fluororesin, as the coating film (11), the adhesion of which is evaluated.

According to the second aspect of the invention, the first cuts (12a) are formed at regular intervals, and the second cuts (12b, 12c) are formed at irregular intervals, thereby achieving the advantages as in the first aspect of the invention.

According to the third aspect of the invention, the intervals between the second cuts (12b, 12c) are gradually changed from one end to the other end. If the narrow and wide intervals between the second cuts (12b, 12c) are mixed, the peeled portions may be also scattered. Thus, it may be difficult to evaluate the adhesion. However, in the present invention, the peeling is concentrated in the portions between the second cuts (12b, 12c), which have the shorter intervals, thereby easily determining the adhesion.

According to the fourth aspect of the invention, the narrower interval between the cuts (12) formed by the cut forming mechanism (20) generates the weaker adhesion of the coating film (11) to the base material (10). The coating film (11) tends to be peeled off in the portion having the narrower interval between the cuts (12) as compared to the portion having the wider interval between the cuts (12). Thus, the intervals between the cuts (12), at which the cells of the coating film (11) are peeled off, are evaluated, thereby quantitatively determining the adhesion of the coating film (11). Consequently, the adhesion of the coating film (11) can be more accurately evaluated as compared to a conventional way. In addition, in the present invention, it is not necessary to adhere the jigs on the base material (10), resulting in the simple measurement.

According to the fifth aspect of the invention, the first cuts (12a) are formed at regular intervals, and the second cuts (12b, 12c) are formed at irregular intervals, thereby achieving the advantages as in the fourth aspect of the invention.

According to the sixth aspect of the invention, the intervals between the second cuts (12b, 12c) are gradually changed from one end to the other end. If the narrow and wide intervals between the second cuts (12b, 12c) are mixed, the peeled portions may be also scattered. Thus, it may be difficult to evaluate the adhesion. However, in the present invention, the peeling is concentrated in the portions between the second cuts (12b, 12c), which have the shorter intervals, thereby easily determining the adhesion.

According to the seventh to ninth aspects of the invention, if the coating having smaller friction resistance is formed on the sliding bearing, the adhesion of such a coating can be easily determined based on the first cuts (12a) formed at regular intervals in the axial direction, and the second cuts (12b, 12c) formed at irregular intervals in the circumferential direction.

### BRIEF DESCRIPTION OF THE DRAWINGS

[FIG. 1] FIG. 1 is a schematic view of a structure of a quantitative cross-cut testing device of an embodiment of the present invention.
[FIG. 2] FIG. 2 is a development view of an inner surface of a work.
[FIG. 3] FIG. 3 is a schematic view of a structure of a quantitative cross-cut testing device, which illustrates a variation of FIG. 1.
[FIG. 4] FIG. 4 is a table showing measurement results of an example.
[FIG. 5] FIG. 5 is a development view of an inner surface of a work of other embodiment.

### DESCRIPTION OF REFERENCE CHARACTERS

- 1: Cross-Cut Testing Device
- 10: Base Material
- 11: Coating Film
- 12: Cut
- 12a: Axial (Vertical) Cut (First Cut)
- 12b: Circumferential (Horizontal) Cut (Second Cut)
- 12c: Spiral Cut (Second Cut)
- 20: Cut Forming Mechanism (Peeling Mechanism)
- 20a: First Cutting Means
- 20b: Second Cutting Means

### DESCRIPTION OF EMBODIMENTS

Embodiments of the present invention will be described in detail hereinafter with reference to the drawings. A cross-cut test of the present invention is referred to as a "quantitative cross-cut test" in the present embodiment.

### <Device Structure>

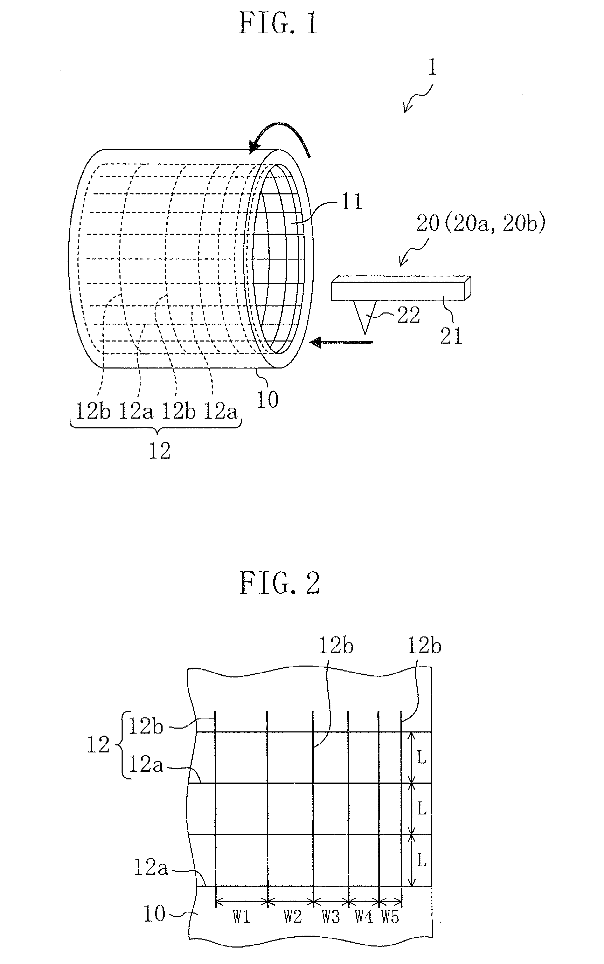

FIG. 1 is a schematic view of a structure of a quantitative cross-cut testing device (1) of the present embodiment. The quantitative cross-cut testing device (1) includes a cut forming mechanism (20) for horizontally and vertically forming cuts (12) in a coating film (11) applied on an inner surface of a sliding bearing (cylindrical base material) to be a work (10). The inner circumferential surface of the work (10) is coated with the coating film (11) as described above. Although not illustrated in the figure, the quantitative cross-cut testing device (1) is configured so that the work (10) rotates about a shaft center.

The cut forming mechanism (20) includes a rod-like body (21) arranged parallel to an axial direction of the work (10); and a cut forming section (22) provided with a blade edge protruding from a tip end of the body (21) in a direction perpendicular thereto. The body (21) is movable in the axial direction, and is movable in a direction perpendicular to the axial direction between points where the blade edge of the cut forming section (22) is pressed against and is retracted from the inner surface of the work (10).

### <Operation>

In the quantitative cross-cut testing device (1), an operation is repeated, in which the body (21) of the cut forming mechanism (20) moves back and forth in the axial direction at a position where the blade edge is slightly cut into the inner surface of the work (10) with the work (10) being stopped, and the work (10) rotates at a predetermined angle. Consequently, first axial cuts (12a) are formed at regular intervals in the coating film (11) (first cutting means (20a)). That is, while intermittently rotating the work (10), the first axial (vertical) cuts (12a) are formed at regular intervals.

Next, the blade edge is slightly cut into the inner surface of the work (10) with the body (21) of the cut forming mechanism (20) being inserted all the way through the work (10), and then second circumferential (horizontal) cuts (12b) are formed by rotating the work (10) at least one revolution. After a single second circumferential cut (12b) is formed, the body (21) of the cut forming mechanism (20) moves so that the blade edge is retracted from the work (10). Subsequently, the body (21) moves back a predetermined distance. The blade edge is again pressed against the inner surface of the work (10) at such a position, and then the work (10) rotates to form another second circumferential cut (12b). As described above, the second circumferential cuts (12b) are intermittently formed. At this point, the distance between the second cuts (12b), over which the body (21) moves back, is gradually shortened, thereby forming the second circumferential cuts (12b) at irregular intervals so that the intervals are gradually narrowed from back to front of the work (10) (second cutting means (20b)).

As described above, a plurality of unequally-spaced second cuts (12b) continuing in the circumferential direction; and a plurality of equally-spaced first cuts (12a) continuing in the axial direction are formed in the work (10). FIG. 2 is a development view of the inner surface of the work (10), and illustrates that the first cuts (12a) in one of the groups of vertical and horizontal cuts (12) are formed at regular intervals (L), and the second cuts (12b) in the other group of vertical and horizontal cuts (12) are formed at irregular intervals (W1-W5).

A peeling process in which a peeling force acts on the coating film (11) after the formation of the cuts (12) is performed by a peeling mechanism (20). In the present embodiment, the peeling mechanism (20) is configured by using the peeling force which naturally acts on the coating film (11) between the second cuts (12b) by narrowing the intervals between the second circumferential cuts (12b) in the formation of the cuts (12). That is, in the quantitative cross-cut testing device (1), the cut forming mechanism (20) also serves as the peeling mechanism (20), and the peeling process is performed at approximately the same time as a cut forming process.

Next, the intervals between the second cuts (12b) of the coating film (11) peeled off by forming the cuts (12) in the coating film (11) as described above are measured. An evaluating process is performed, in which adhesion of the coating film (11) is evaluated based on a peeling state of the coating film (11). At this point, the intervals between the second cuts (12b) may be measured by using, e.g., a microscope.

### <Advantages of Embodiment>

According to the present embodiment, the intervals between the second cuts (12b) formed in the coating film (11) on the work (10) are differentiated, thereby changing a tendency of the coating film (11) to be peeled off (adhesion). Consequently, the coating film (11) tends to be peeled off in a portion having a narrower interval between the second cuts (12b) as compared to a portion having a wider interval between the second cuts (12b). Thus, the intervals between the second cuts (12b), at which the cells of the coating film (11) are peeled off, are evaluated, thereby quantitatively determining the adhesion of the coating film (11). Consequently, the adhesion of the coating film (11) can be more accurately evaluated as compared to a conventional way. In addition, in the present embodiment, it is not necessary to adhere jigs or adhesive tapes on the work (10), resulting in a particularly simple measurement.

In the present embodiment, the intervals between the second cuts (12b) are gradually changed from one end to the other end. If narrow and wide intervals between the second cuts (12b) are mixed, peeled portions are also scattered. Thus, it may be difficult to evaluate the adhesion. However, in the present embodiment, the peeling is caused in portions between the second cuts (12b), which have the shorter intervals, thereby easily determining the adhesion. That is, a smaller cell where the coating film (11) is peeled off, generates higher adhesion of the coating film (11) to the work (10).

### <Variations of Embodiment>

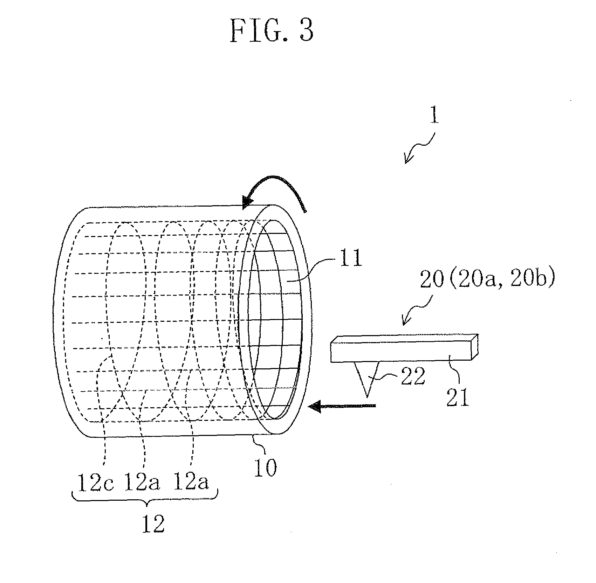

In the example illustrated in FIG. 1, the second circumferential cuts (12b) are a plurality of second cuts (12b) which are arranged parallel to each other, and which are discontinuous in the axial direction. However, as illustrated in FIG. 3, instead of forming the second cuts (12b) parallel to each other in the circumferential direction, and discontinuously forming them in the axial direction, a single second spiral cut (12c) which is continuous in the circumferential and axial directions, and a torsion angle of which is gradually changed may be formed so as to narrow the intervals between the portions of the second cut (12c) from one end to the other end.

In addition to achieve the advantages as in the foregoing embodiment, this continuously forms the second circumferential cut (12c), thereby facilitating the working processes.

### <Other Embodiments>

The foregoing embodiment may have the following structures.

In the foregoing embodiment, the example has been described, where the sliding bearing is used as the work (10). However, the work (10) (base material) does not necessarily have the cylindrical shape, and the present invention may be applied in order to evaluate adhesion of a coating film (11) formed on a tabular or concave-convex base material. Even if the present invention is applied to the cylindrical work (10), circumferential cuts may be formed at regular intervals, and axial cuts may be formed at irregular intervals.

It is preferred that the cells are changed from a square shape to a rectangular shape. However, cells may be formed in a diamond shape to change the intervals between the cuts (12).

In the foregoing embodiment, the cut forming mechanism (20) serves as the peeling mechanism (20) in order not to purposely peel off the coating film (11) by adhesive tapes, etc. Such structure is particularly preferred when material such as fluororesin to which a tape is hardly adhered is used as the coating film (11). However, depending on materials of the coating film (11), a peeling mechanism (20) only for providing a peeling force by using adhesive tapes, etc. may be provided to perform a peeling process.

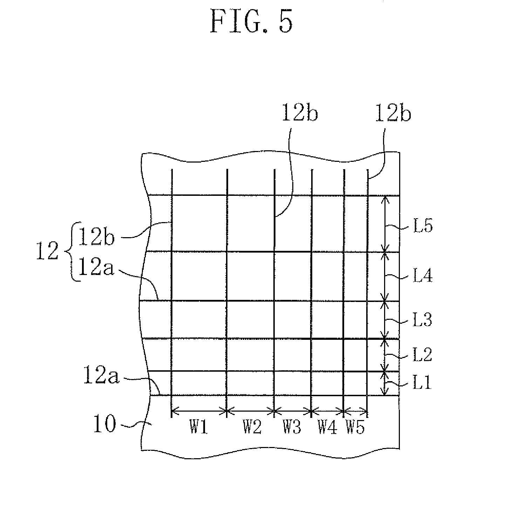

In the foregoing embodiment, the first cuts (12a) are formed at regular intervals, and the second cuts (12b) are formed at irregular intervals. However, as illustrated in FIG. 5, the first cuts (12a) are not necessarily formed at regular intervals, and may be formed at irregular intervals (L1-L5). In such a case, the adhesion of the coating film (11) can be also quantitatively evaluated based on the intervals between the cuts (12a, 12b), at which the cells of the coating film (11) are peeled off. FIG. 5 illustrates an example where the first cuts (12a) and the second cuts (12b) are formed so that the intervals therebetween are gradually changed from one end to the other end. However, the way to change the intervals may be altered as necessary. In such a case, the adhesion of the coating film (11) can be also quantitatively determined by measuring the intervals between the cuts (12a, 12b) in the evaluating process.

The foregoing embodiments have been set forth merely for purposes of preferred examples in nature, and are not intended to limit the applications, and use of the invention.

### Examples

In a comparative example (A), a coating film (11) made of polyamide imide (PAI) and polytetrafluoroethylene (PTFE) is applied to an inner circumferential surface of a base material (10) formed by applying a manganese phosphate film coating treatment to cylindrical carbon steel (S45C) material, followed by burning thereof. The base material with the prepared coating is used as a test specimen (A).

In a comparative example (B), a coating film (11) made of PAI and PTFE is applied to an inner circumferential surface of a base material (sintered metal material) (10) formed by sintering iron powder, followed by burning thereof. The base material with the prepared coating is used as a test specimen (B).

In an example, a stem treatment is applied to a base material (sintered metal material) (10) formed by sintering iron powder, and then a coating film (11) made of PAI and PTFE is applied to an inner circumferential surface of the base material (10), followed by burning thereof. The base material with the prepared coating is used as a test specimen (C). In such a case, an oxide film is formed on a surface of the base material (10) by the steam treatment as described later, and the coating film (11) made of PAI and PTFE is formed thereon.

The test specimens (A), (B), and (C) are examined by both of the quantitative cross-cut test of the present invention, and a cross-cut test according to JIS K 5600-5-6, thereby measuring adhesion of the coating film (11). Specific examples such as sample preparation, testing devices, and.measurement conditions will be described here. Note that vertical and horizontal intervals between cuts (12) are 1 mm in the cross-cut technique according to JIS K 5600-5-6.

### <Sample Preparation>

Material equivalent to Japan Powder Metallurgy Association (JPMA) SMF 4040 is used as the sintered material. The base material is formed in a cylindrical shape with an inner diameter ϕ of 34 mm, an outer diameter ϕ of 44 mm, and a length of 30 mm. Such dimensions are an example, and may be varied.

A composition of the coating film contains PTFE in an amount of 20-45% by mass. The coating film may contain third and fourth fillers such as fluoride and alumina.

The coating film is formed by a coating technique such as a spray technique and a dispenser technique. The thickness upon the burning is 100-150 µm, and the thickness is reduced to 40-60 µm by a cutting. The burning is performed at 200-300°C for 30-60 minutes.

As for the test specimen (C) of the example, the steam treatment is performed in order to form an oxide film on a surface of the sintered material. Specifically, the sintered material is heated within a predetermined temperature range (e.g., 500-560°C) in a water-vapor atmosphere furnace, thereby forming a black oxide film made of Fe₃O₄ with a predetermined uniform thickness (approximately several microns). The sintered material is porous, and the oxide film having the uniform thickness is also formed on inner surfaces of microscopic holes exposed in the surface of the sintered material. The coating film (11) made of PAI and PTFE is formed thereon.

### <Testing Devices, Instruments, and Test Specimens>

It is necessary that a cutting tool (cut forming section) (22) of a testing device (1) has a blade edge in a good condition. In order to form cuts at regular intervals, a regular-interval spacer with a guide may be used when using a single cutting tool (22).

When removing a coating film (11) with no adhesion force, adhesive tapes may be used (e.g., adhesive tapes having an adhesion strength of 10±1N for each 25 mm of width). An approximately 100-300 power optical microscope may be used as an observation instrument used in an evaluating process.

Shapes of the test specimens are not defined. However, it is desirable to perform a test in three different portions which are more than 5 mm apart from ends of the test specimen. The test specimen having the uniform thickness is desirable.

### <Test Conditions>

As described above, the test is performed in at least three different portions of the test specimen, unless otherwise specified, at a temperature of 23±2°C and a relative humidity of 50±5%. In addition, unless otherwise specified, the test specimen is cured at a temperature of 23±2°C and a relative humidity of 50±5% for at least 16 hours immediately before the test.

Cuts are formed in the test specimen at an interval of 1 mm in an X direction of a grid pattern, and at intervals of 5-0.1 mm in a Y direction. This forms 4 cuts in the X direction, and 51 cuts in the Y direction, resulting in a grid having the total of 150 cells.

### <Test Procedures>

First, the test specimen is fixed with a vise. Next, cuts are manually formed according to specified procedures. Note that a blade portion is checked before the test, and is exchanged to maintain its condition.

The cutting tool is held so that the blade is perpendicular to a surface of the test specimen. Uniform pressure is added to the cutting tool, and the specified number of cuts are formed in the film-coating portion at a constant cutting rate by using a suitable spacer as necessary.

The cuts are formed in the X and Y directions at the above-described intervals. All of the cuts should reach the surface of the base material through the coating film. If it is difficult to form the cuts at the interval of 0.1 mm, cuts may be formed without precise measurements so that the intervals therebetween gradually becomes narrower, followed by precisely measuring such intervals by a magnifying glass.

Adhesive tapes may be used in order to remove the coating film with no adhesion force. The adhesive tapes may be kept for observation.

### <Evaluation Method of Measurement Results>

Measurement results are evaluated immediately after the coating film with no adhesion is removed. At this point, the peeled coating film is observed from above by using the observation instrument. Intervals where the cells of the coating film are peeled off, and intervals where the cells of the coating film are not peeled off are quantified. Two numerical values are used as necessary to obtain the measurement results. A shorter interval in the peeled portion generates higher adhesion.

### <Measurement Results>

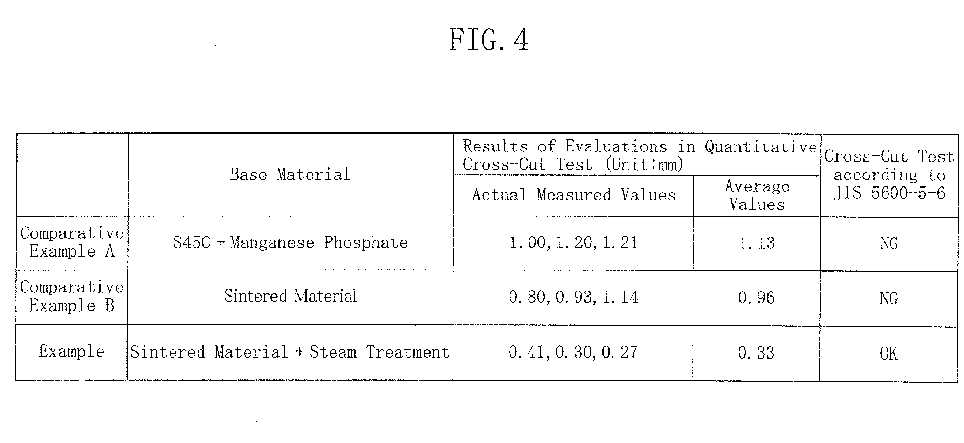

The measurement results are indicated in a table of FIG. 4. Peeling widths in the quantitative cross-cut test as shown in the table are measured by using a digital microscope. Each numerical value is an average value of three measurements.

As shown in the table, the quantitative cross-cut test of the present invention results in the test specimen (C) in the example having the peeling width narrower than those of the test specimen (A) and the test specimen (B) in the comparative examples. Thus, such results show stronger adhesion of the coating film (11) to the test specimen (C), and the adhesion strength as compared to the test specimen (A) and the test specimen (B). The cross-cut technique according to JIS K 5600-5-6 results in the peeling being caused in the test specimens (A) and (B), and the peeling being not caused in the test specimen (C). Thus, such results show the adhesion strength as in the quantitative cross-cut test of the present invention. However, the cross-cut technique according to JIS K 5600-5-6 can only determine the results as "NG (with peeling)" or "OK (without peeling)," whereas the quantitative cross-cut test of the present embodiment can measure the peeling width to quantify and measure the adhesion.

### INDUSTRIAL APPLICABILITY

As described above, the present invention is useful for device and method for obtaining quantitative measurement results in a cross-cut test for measuring coating adhesion to a base material.

## Claims

1. A cross-cut testing method sequentially performing a cut forming process for vertically and horizontally forming cuts (12) in a coating film (11) applied to a base material (10); a peeling process for making a peeling force act on the coating film (11) after the formation of the cuts (12); and an evaluating process for evaluating adhesion of the coating film (11) based on a peeling state of the coating film (11) after the peeling process, the cross-cut testing method **characterized in that**:
the cut forming process includes forming first cuts (12a) in one of the groups of vertical and horizontal cuts at regular or irregular intervals, and forming second cuts (12b, 12c) in the other group of vertical and horizontal cuts at irregular intervals.

2. The cross-cut testing method of claim 1, wherein
the cut forming process includes forming the first cuts (12a) at regular intervals, and for forming the second cuts (12b, 12c) at irregular intervals.

3. The cross-cut testing method of claim 1 or 2, wherein
the cut forming process includes forming the second cuts (12b, 12c) so that the intervals are gradually increased or decreased from one end to the other end.

4. A cross-cut testing device for performing a cross-cut test for a coating film (11), which includes a cut forming mechanism (20) for vertically and horizontally forming cuts (12) in the coating film (11) applied to a base material (10); and a peeling mechanism (20) for making a peeling force act on the coating film (11) in which the cuts (12) are formed, the cross-cut testing device **characterized in that**:
the cut forming mechanism (20) includes first cutting means (20a) for forming first cuts (12a) in one of the groups of vertical and horizontal cuts at regular or irregular intervals; and second cutting means (20b) for forming second cuts (12b, 12c) in the other group of vertical and horizontal cuts at irregular intervals.

5. The cross-cut testing device of claim 4, wherein
the first cutting means (20a) forms the first cuts (12a) at regular intervals.

6. The cross-cut testing device of claim 4 or 5, wherein
the second cutting means (20b) forms the second cuts (12b, 12c) in the coating film (11) so that the intervals are gradually increased or decreased from one end to the other end.

7. The cross-cut testing device of claim 5 or 6, wherein
the cut forming mechanism (20) is intended to form the cuts in the coating film (11) formed on the cylindrical base material (10);
the first cutting means (20a) forms the first cuts (12a) in an axial direction at regular intervals; and
the second cutting means (20b) forms the second cuts (12b, 12c) in a circumferential direction at irregular intervals.

8. The cross-cut testing device of claim 7, wherein
the second cutting means (20b) forms a plurality of second cuts (12b) which are continuous in the circumferential direction, and which are discontinuous in the axial direction.

9. The cross-cut testing device of claim 7, wherein
the second cutting means (20b) forms a second cut (12c) which is a single spiral continuous in the circumferential and axial directions, and a torsion angle of which is gradually changed.
